(19) Europäisches Patentamt
European Patent Office
Office européen des brevets

(11) Veröffentlichungsnummer: **0 142 111**
**A2**

(12) # EUROPÄISCHE PATENTANMELDUNG

(21) Anmeldenummer: **84113275.6**

(22) Anmeldetag: **05.11.84**

(51) Int. Cl.[4]: **C 07 C 155/02**
C 07 D 295/20, C 07 D 207/06
C 07 D 211/16, C 07 D 265/30
A 01 N 47/10

(30) Priorität: **17.11.83 DE 3341515**

(43) Veröffentlichungstag der Anmeldung:
**22.05.85 Patentblatt 85/21**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Kühle, Engelbert, Dr.**
**Bodelschwinghstrasse 42**
**D-5060 Bergisch Gladbach 2(DE)**

(72) Erfinder: **Klauke, Erich, Dr.**
**Eichendorffweg 8**
**D-5068 Odenthal(DE)**

(72) Erfinder: **Eue, Ludwig, Dr.**
**Paul-Klee-Strasse 36**
**D-5090 Leverkusen(DE)**

(72) Erfinder: **Schmidt, Robert R., Dr.**
**Im Waldwinkel 110**
**D-5060 Bergisch-Gladbach 2(DE)**

(54) **Fluorierte Thiolcarbamate.**

(57) Neue fluorierte Thiolcarbamate der Formel

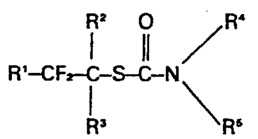

(I)

in welcher

$R^1$ für Alkyl, Cycloalkyl oder gegebenenfalls substituiertes Aryl steht,

$R^2$ und $R^3$ unabhängig voneinander für Wasserstoff oder Alkyl stehen oder

$R^1$ und $R^2$ gemeinsam für eine Alkylenkette stehen und

$R^4$ und $R^5$ unabhängig voneinander für Wasserstoff, Alkyl, Alkenyl, Alkinyl, Alkoxyalkyl, Alkylthioalkyl, Cycloalkyl oder einen gesättigten oder ungesättigten, gegebenenfalls substituierten heterocyclischen Ring mit 5 bis 8 Ringgliedern stehen, oder

$R^4$ und $R^5$ gemeinsam mit dem angrenzenden Stickstoffatom für einen gegebenenfalls substituierten, gesättigten oder ungesättigten heterocyclischen Ring mit 5 bis 8 Ringgliedern stehen,

ein Verfahren zur Herstellung dieser neuen Stoffe und deren Verwendung als Herbizide.

EP 0 142 111 A2

Croydon Printing Company Ltd.

BAYER AKTIENGESELLSCHAFT 5090 Leverkusen, Bayerwerk

Dü-klu/c
Ia

# Fluorierte Thiolcarbamate

Die Erfindung betrifft neue fluorierte Thiolcarbamate, ein Verfahren zu deren Herstellung und deren Verwendung als Herbizide.

Es ist bereits bekannt geworden, daß zahlreiche Thiolcarbamate herbizide Eigenschaften besitzen (vgl. US-PS 3 198 786). So läßt sich zum Beispiel Hexahydro-1-H-azepin-1-carbamidsäure-thiolethylester zur Unkrautbekämpfung verwenden. Die selektive herbizide Wirksamkeit dieses Stoffes ist jedoch vor allem bei niedrigen Aufwandmengen nicht immer ausreichend.

Es wurden jetzt neue fluorierte Thiolcarbamate der Formel

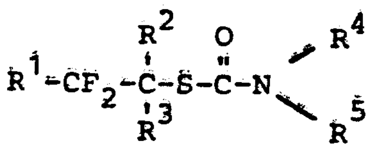

(I)

in welcher

$R^1$ für Alkyl, Cycloalkyl oder gegebenenfalls substituiertes Aryl steht,

$R^2$ und $R^3$ unabhängig voneinander für Wasserstoff oder Alkyl stehen oder

$R^1$ und $R^2$ gemeinsam für eine Alkylenkette stehen und

$R^4$ und $R^5$ unabhängig voneinander für Wasserstoff, Alkyl, Alkenyl, Alkinyl, Alkoxyalkyl, Alkylthioalkyl, Cycloalkyl oder einen gesättigten oder ungesättigten, gegebenenfalls substituierten heterocyclischen Ring mit 5 bis 8 Ringgliedern stehen, oder

$R^4$ und $R^5$ gemeinsam mit dem angrenzenden Stickstoffatom für einen gegebenenfalls substituierten, gesättigten oder ungesättigten heterocyclischen Ring mit 5 bis 8 Ringgliedern stehen,

gefunden.

Weiterhin wurde gefunden, daß man die neuen fluorierten Thiolcarbamate der Formel (I) erhält, wenn man fluorierte Thiokohlensäureesterfluoride der Formel

$$R^1-CF_2-\overset{\displaystyle R^2}{\underset{\displaystyle R^3}{\overset{|}{\underset{|}{C}}}}-S-\underset{\displaystyle O}{\underset{\|}{C}}-F \qquad \text{(II)}$$

in welcher

0142111

$R^1$, $R^2$ und $R^3$ die oben angegebene Bedeutung haben,

mit Verbindungen der Formel

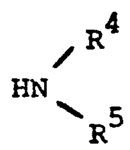

(III)

in welcher

$R^4$ und $R^5$ die oben angegebene Bedeutung haben,

gegebenenfalls in Gegenwart eines Verdünnungsmittels sowie gegebenenfalls in Gegenwart eines Säurebindemittels umsetzt.

Schließlich wurde gefunden, daß die neuen fluorierten Thiolcarbamate der Formel (I) sehr gute herbizide Eigenschaften besitzen und vor allem zur selektiven Unkrautbekämpfung geeignet sind.

Es ist als äußerst überraschend zu bezeichnen, daß die erfindungsgemäßen Stoffe eine bessere selektive herbizide Wirksamkeit zeigen als der Hexahydro-1-H-azepin-1-carbamidsäure-thiolethylester, welches ein konstitutionell ähnlicher Wirkstoff analoger Wirkungsrichtung ist.

Die erfindungsgemäßen Wirkstoffe sind durch die Formel (I) allgemein definiert. In dieser Formel steht $R^1$ vorzugsweise für geradkettiges oder verzweigtes Alkyl mit

0142111

1 bis 12 Kohlenstoffatomen, Cycloalkyl mit 3 bis 8 Kohlenstoffatomen oder für Phenyl, welches ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen, Alkylthio mit 1 bis 4 Kohlenstoffatomen, Halogenalkylthio mit 1 bis 4 Kohlenstoffatomen und 1 bis 5 Halogenatomen und/oder Nitro. Die Reste $R^2$ und $R^3$ stehen unabhängig voneinander vorzugsweise für Wasserstoff oder geradkettiges oder verzweigtes Alkyl mit 1 bis 4 Kohlenstoffatomen. Die Reste $R^1$ und $R^2$ stehen auch gemeinsam vorzugsweise für eine Alkylenkette mit 3 bis 5 Kohlenstoffatomen. Die Reste $R^4$ und $R^5$ stehen unabhängig voneinander vorzugsweise für Wasserstoff, Alkyl mit 1 bis 4 Kohlenstoffatomen, Alkenyl mit 2 bis 4 Kohlenstoffatomen, Alkinyl mit 2 bis 4 Kohlenstoffatomen, Alkoxyalkyl mit 1 bis 4 Kohlenstoffatomen im Alkoxyteil und 1 bis 4 Kohlenstoffatomen im Alkylteil, Alkylthioalkyl mit 1 bis 4 Kohlenstoffatomen im Alkylthioteil und 1 bis 4 Kohlenstoffatomen im Alkylteil, Cycloalkyl mit 3 bis 8 Kohlenstoffatomen oder für einen gesättigten oder ungesättigten, gegebenenfalls durch Halogen, Alkyl mit 1 bis 4 Kohlenstoffatomen, Alkoxy mit 1 bis 4 Kohlenstoffatomen und/oder Phenyl substituierten heterocyclischen Ring mit 5 bis 8 Ring-Gliedern und 1 bis 3 Heteroatomen bzw. Heteroatom-Gruppen, wie Sauerstoff, Stickstoff, Schwefel, $SO_2$, SO und/oder NR, worin R für Wasserstoff oder Alkyl mit 1 bis 4 Kohlenstoffatomen steht. Außerdem stehen die Reste $R^4$ und $R^5$ auch gemeinsam mit dem angrenzenden Stickstoffatom vorzugsweise für einen gesättigten oder ungesättigten, gegebenenfalls durch Halogen, Alkyl mit

1 bis 4 Kohlenstoffatomen, Alkoxy mit 1 bis 4 Kohlenstoffatomen und/oder Phenyl substituierten Heterocyclus mit 5 bis 8 Ringgliedern und 1 bis 3 Heteroatomen bzw. Heteroatom-Gruppen, wie Sauerstoff, Stickstoff, Schwefel, SO, $SO_2$ und/oder NR, worin R für Wasserstoff oder Alkyl mit 1 bis 4 Kohlenstoffatomen steht.

Besonders bevorzugt sind diejenigen Verbindungen der Formel (I), in denen $R^1$ für geradkettiges oder verzweigtes Alkyl mit 1 bis 8 Kohlenstoffatomen, Cycloalkyl mit 3 bis 7 Kohlenstoffatomen oder für Phenyl steht, welches ein- oder mehrfach, gleichartig oder verschieden substituiert sein kann durch Fluor, Chlor, Halogenalkyl mit 1 oder 2 Kohlenstoffatomen und 1 bis 3 Fluor- oder Chloratomen, Alkyl mit 1 bis 3 Kohlenstoffatomen, Halogenalkoxy mit 1 oder 2 Kohlenstoffatomen und 1 bis 3 Fluor- oder Chloratomen, Alkoxy mit 1 bis 3 Kohlenstoffatomen, Alkylthio mit 1 bis 3 Kohlenstoffatomen, Halogenalkylthio mit 1 oder 2 Kohlenstoffatomen und 1 bis 3 Fluor- oder Chloratomen und/oder Nitro,

$R^2$ und $R^3$ unabhängig voneinander für Wasserstoff oder geradkettiges oder verzweigtes Alkyl mit 1 bis 3 Kohlenstoffatomen stehen,

$R^1$ und $R^2$ gemeinsam für eine Alkylenkette mit 3 oder 4 Kohlenstoffatomen stehen,

$R^4$ und $R^5$ unabhängig voneinander für Wasserstoff, Alkyl mit 1 bis 3 Kohlenstoffatomen, Alkenyl mit 2 oder 3 Kohlenstoffatomen, Alkinyl mit 2 oder 3 Kohlenstoffatomen, Alkoxyalkyl mit 1 bis 3 Kohlenstoffatomen im Alkoxyteil und 1 bis 3 Kohlenstoffatomen im Alkylteil, Alkylthioalkyl mit 1 bis 3 Kohlenstoffatomen im Alkylthioteil und 1 bis 3 Kohlenstoffatomen im Alkylteil,

Cycloalkyl mit 3 bis 7 Kohlenstoffatomen oder für einen gesättigten oder ungesättigten, gegebenenfalls durch Fluor, Chlor, Alkyl mit 1 bis 3 Kohlenstoffatomen, Alkoxy mit 1 bis 3 Kohlenstoffatomen und/oder Phenyl substituierten Heterocyclus mit 5 bis 8 Ringgliedern und 1 bis 3 Heteroatomen bzw. Heteroatom-Gruppen, wie Sauerstoff, Schwefel, Stickstoff, SO, $SO_2$ und/oder NR stehen, worin R für Wasserstoff, Methyl oder Ethyl steht, oder

$R^4$ und $R^5$ gemeinsam mit dem angrenzenden Stickstoffatom für einen gesättigten oder ungesättigten, gegebenenfalls durch Fluor, Chlor, Alkyl mit 1 bis 3 Kohlenstoffatomen, Alkoxy mit 1 bis 3 Kohlenstoffatomen und/oder Phenyl substituierten Heterocyclus mit 5 bis 8 Ringgliedern und 1 bis 3 Heteroatomen bzw. Heteroatom-Gruppen, wie Sauerstoff, Schwefel, Stickstoff, SO, $SO_2$ und/oder NR stehen, worin R für Wasserstoff, Methyl oder Ethyl steht.

Als Beispiele für heterocyclische Reste seien in diesem Zusammenhang genannt: Pyrrolyl, Pyrrolidinyl, Furanyl, Tetrahydrofuranyl, Thiophenyl, Tetrahydrothiophenyl, Piperidyl, Pyridinyl, Pyrazolyl, Imidazol-1-yl, 1,2,4-Triazol-1-yl, 1,3,4-Triazol-1-yl, Morpholinyl, Thiamorpholinyl, Thiamorpholinyldioxid, Hexamethyleniminyl, Heptamethyleniminyl, Piperazinyl, Thiazolyl, Oxazolyl und Isoxazolyl, wobei jeder dieser Reste ein- oder mehrfach, gleichartig oder verschieden substituiert sein kann durch Fluor, Chlor, Methyl, Ethyl, Methoxy, Ethoxy und/oder Phenyl.

Verwendet man 2,2-Difluorpropanthiol-kohlensäureesterfluorid und Di-n-propylamin als Ausgangsstoffe, so kann der Verlauf des erfindungsgemäßen Verfahrens durch das folgende Formelschema veranschaulicht werden:

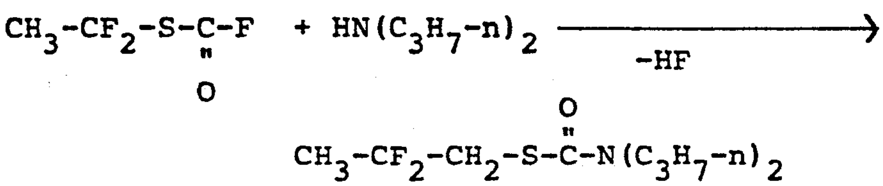


Die bei dem erfindungsgemäßen Verfahren als Ausgangsstoffe benötigten fluorierten Thiokohlensäureesterfluoride sind durch die Formel (II) allgemein definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden.

Die fluorierten Thiokohlensäureesterfluoride der Formel (II) sind bisher noch nicht bekannt. Sie lassen sich jedoch herstellen, indem man ß-Keto-trihalogenmethyl-thioether der Formel

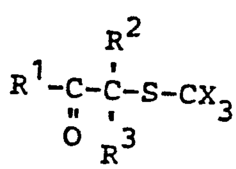

(IV)

in welcher

$R^1$, $R^2$ und $R^3$ die oben angegebene Bedeutung haben und
X für Fluor oder Chlor steht, wobei mindestens ein X für Chlor steht,

mit wasserfreier Flußsäure, gegebenenfalls in Gegenwart eines Verdünnungsmittels, umsetzt.

Die bei dem Verfahren zur Herstellung der fluorierten Thiokohlensäureesterfluoride der Formel (II) als Ausgangsstoffe benötigten ß-Keto-trihalogenmethyl-thioether sind durch die Formel (IV) allgemein definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. X steht für Fluor oder Chlor, wobei jedoch mindestens ein X für Chlor steht.

Als Beispiele für ß-Keto-trihalogenmethyl-thioether der Formel (IV) seien die in den beiden folgenden Tabellen aufgeführten Stoffe erwähnt.

Tabelle 1

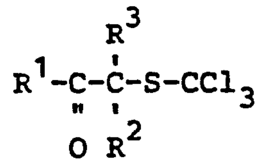

(IVa)

| $R^1$ | $R^2$ | $R^3$ |
|---|---|---|
| $CH_3$ | $CH_3$ | $CH_3$ |
| $C_2H_5$ | $CH_3$ | H |
| $C_3H_7$-n | $CH_3$ | H |
| n-$C_4H_9$- | $CH_3$ | H |
| n-$C_5H_{11}$ | H | H |
| n-$C_6H_{12}$ | H | H |
| n-$C_{12}H_{25}$ | H | H |
| 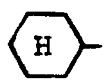 | H | H |
| 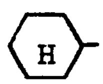 | H | H |
| 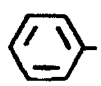 | H | H |
| 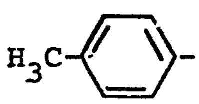 | H | H |
| 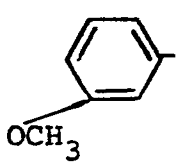 | H | H |
| 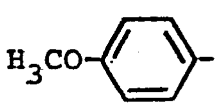 | H | H |

Tabelle 1 (Fortsetzung)

| $R^1$ | $R^2$ | $R^3$ |
|---|---|---|
| 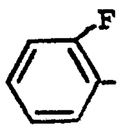 | H | H |
| 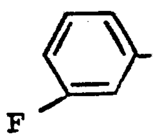 | H | H |
| 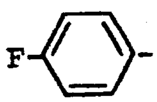 | H | H |
| 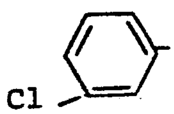 | H | H |
| 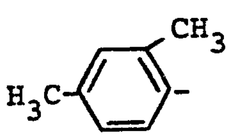 | H | H |
| 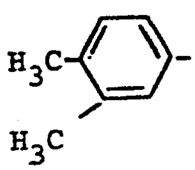 | H | H |
| 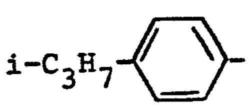 | H | H |
| 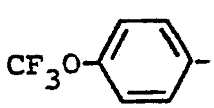 | H | H |

Tabelle 1 (Fortsetzung)

| $R^1$ | $R^2$ | $R^3$ |
|---|---|---|
| 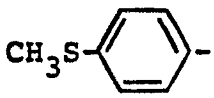 | H | H |
| 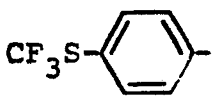 | H | H |

Tabelle 2

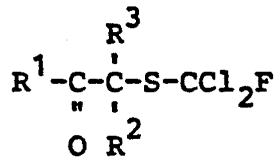

(IVb)

| $R^1$ | $R^2$ | $R^3$ |
|---|---|---|
| $CH_3$ | $CH_3$ | $CH_3$ |
| $C_2H_5$ | $CH_3$ | H |
| $C_3H_7$-n | $CH_3$ | H |
| n-$C_4H_9$- | $CH_3$ | H |
| n-$C_5$-$H_{11}$ | H | H |
| n-$C_6H_{12}$ | H | H |
| n-$C_{12}H_{25}$ | H | H |
| 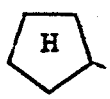 | H | H |
| 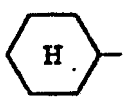 | H | H |
| 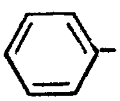 | H | H |
| 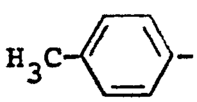 | H | H |
| 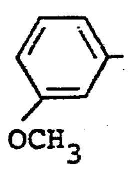 | H | H |
| 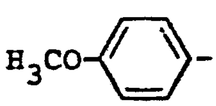 | H | H |

Tabelle 2 (Fortsetzung)

| $R^1$ | $R^2$ | $R^3$ |
|---|---|---|
| 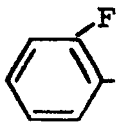 | H | H |
| 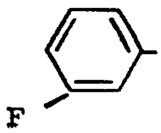 | H | H |
| 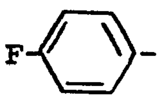 | H | H |
| 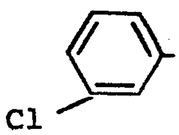 | H | H |
| 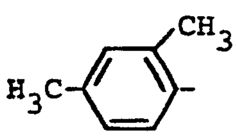 | H | H |
| 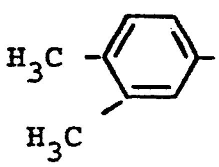 | H | H |
| 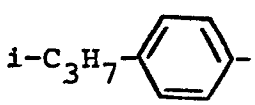 | H | H |
| 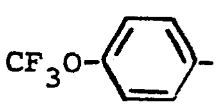 | H | H |

Tabelle 2 (Fortsetzung)

| $R^1$ | $R^2$ | $R^3$ |
|---|---|---|
| 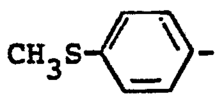 | H | H |
| 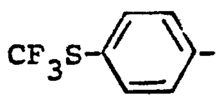 | H | H |
| 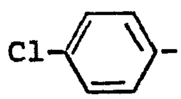 | H | H |
| 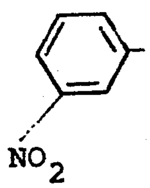 | H | H |
| 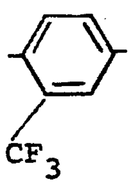 | H | H |

Die ß-Keto-trihalogenmethyl-thioether der Formel (IV) sind teilweise bekannt (vgl. US-PS 3 937 738 und E. Kühle "The Chemistry of the Sulfenic Acids", Georg Thieme Publ. Stuttgart, 1973, Seite 95).

Die ß-Keto-trihalogenmethyl-thioether der Formel

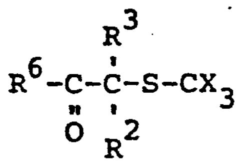

(IVc)

in welcher

$R^2$, $R^3$ und X die oben angegebene Bedeutung haben und
$R^6$ für gegebenenfalls substituiertes Aryl steht,

sind bisher noch nicht beschrieben worden.

In den Verbindungen der Formel (IVc) steht $R^6$ vorzugsweise für Phenyl, welches ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 4 Halogenatomen, Alkylthio mit 1 bis 4 Kohlenstoffatomen, Halogenalkylthio mit 1 bis 4 Kohlenstoffatomen und 1 bis 5 Halogenatomen und/oder Nitro.

Besonders bevorzugt sind diejenigen Verbindungen der Formel (IVc), in denen $R^6$ für Phenyl steht, welches ein- oder mehrfach, gleichartig oder verschieden substituiert sein kann durch Fluor, Chlor, Alkyl mit 1 bis 3 Kohlenstoffatomen, Halogenalkyl mit 1 oder 2 Kohlenstoffatomen und 1 bis 3 Fluor- oder Chloratomen, Alkoxy

mit 1 bis 3 Kohlenstoffatomen, Halogenalkoxy mit 1 oder 2 Kohlenstoffatomen und 1 bis 3 Fluor- oder Chloratomen, Alkylthio mit 1 bis 3 Kohlenstoffatomen, Halogenalkylthio mit 1 oder 2 Kohlenstoffatomen und 1 bis 3 Fluor- oder Chloratomen und/oder Nitro. Die Reste $R^2$, $R^3$ und X haben vorzugsweise diejenigen Bedeutungen, die oben bereits als bevorzugt beziehungsweise als besonders bevorzugt genannt wurden.

Die Verbindungen der Formel (IVc) lassen sich herstellen, indem man Ketone der Formel

$$\begin{array}{r} R^3 \\ R^6\text{-CO-}\overset{|}{\underset{|}{C}}\text{-H} \\ R^2 \end{array} \qquad \text{(V)}$$

in welcher

$R^2$, $R^3$ und $R^6$ die oben angegebene Bedeutung haben,

mit Trihalogenmethyl-sulfenyl-chloriden der Formel

$$Cl\text{-}S\text{-}CX_3 \qquad \text{(VI)}$$

in welcher

X die oben angegebene Bedeutung hat,

gegebenenfalls in Gegenwart eines Verdünnungsmittels, wie zum Beispiel Chloroform, bei Temperaturen zwischen 0°C und 100°C umsetzt. Die Aufarbeitung erfolgt nach üblichen Methoden.

Auch die ß-Keto-trihalogenmethyl-thioether der Formel

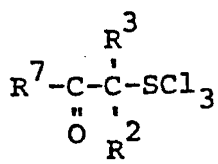

(IVd)

in welcher

$R^2$ und $R^3$ die oben angegebene Bedeutung haben und
$R^7$ für Alkyl oder Cycloalkyl steht,

sind neu.

In den Verbindungen der Formel (IVd) steht $R^7$ vorzugsweise für geradkettiges oder verzweigtes Alkyl mit 1 bis 12 Kohlenstoffatomen oder für Cycloalkyl mit 3 bis 8 Kohlenstoffatomen. Besonders bevorzugt sind diejenigen Verbindungen der Formel (IVd), in denen $R^7$ für geradkettiges oder verzweigtes Alkyl mit 1 bis 8 Kohlenstoffatomen oder für Cycloalkyl mit 3 bis 7 Kohlenstoffatomen steht.

Die Verbindungen der Formel (IVd) lassen sich herstellen, indem man Ketone der Formel

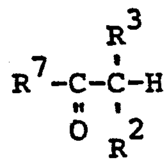

(VII)

in welcher

$R^2$, $R^3$ und $R^7$ die oben angegebene Bedeutung haben,

mit Trichlormethyl-sulfenyl-chlorid der Formel

$$Cl\text{-}S\text{-}CCl_3 \qquad \text{(VIa)}$$

gegebenenfalls in Gegenwart eines inerten Verdünnungsmittels bei Temperaturen zwischen 0°C und 100°C umsetzt. Die Aufarbeitung erfolgt nach üblichen Methoden.

Bei dem Verfahren zur Herstellung der fluorierten Thiokohlensäureesterfluoride der Formel (II) dient wasserfreie Flußsäure als Fluorierungsmittel.

Die oben beschriebene Umsetzung zur Herstellung der Verbindungen der Formel (II) kann gegebenenfalls in Gegenwart eines Verdünnungsmittels durchgeführt werden, das unter den Reaktionsbedingungen inert ist. Vorzugsweise verwendbare Solventien sind hierbei chlorierte Kohlenwasserstoffe, wie Methylenchlorid, Chloroform und Chlorbenzol.

Die Reaktionstemperatur kann bei der Durchführung des obigen Verfahrens zur Herstellung der Verbindungen der

Formel (II) innerhalb eines bestimmten Bereiches variiert werden. Im allgemeinen arbeitet man bei Temperaturen zwischen -20°C und +100°C, vorzugsweise zwischen -10°C und +60°C.

Die Umsetzung zur Herstellung der Verbindungen der Formel (II) kann sowohl unter Normaldruck als auch unter erhöhtem Druck vorgenommen werden. Der Überdruck kann dabei bis zu 6 bar betragen.

Die Umsetzung zur Herstellung der Verbindungen der Formel (II) wird in Apparaturen durchgeführt, die üblicherweise für Reaktionen zum Austausch von Chlor gegen Fluor gebräuchlich sind.

Bei der Durchführung des obigen Verfahrens zur Synthese der fluorierten Thiolkohlensäureesterfluoride der Formel (II) geht man im allgemeinen so vor, daß man die wasserfreie Flußsäure vorlegt und die ß-Keto-trihalogenmethyl-thioether der Formel (IV) bei niedriger Temperatur zugibt. Dabei wird im allgemeinen auf die Verwendung eines zusätzlichen Lösungsmittels verzichtet. Sollen jedoch kristalline Ausgangssubstanzen zugegeben werden, so geschieht dieses zweckmäßigerweise so, daß man die betreffenden Substanzen in einem unter den Reaktionsbedingungen inerten Solvens gelöst hinzugibt.

Die Umsetzung verläuft im allgemeinen bereits bei niedrigen Temperaturen recht zügig; sie ist unter drucklosen Bedingungen zumeist schon innerhalb weniger Stunden beendet. Verwendet man relativ reaktionsträge Ausgangsmaterialien, so arbeitet man zweckmäßigerweise unter erhöhtem Druck bei etwas höheren Temperaturen. Dabei ist dafür zu sorgen, daß der frei werdende Chlorwasserstoff über ein regelbares Ventil entspannt werden kann.

Die Menge der einzusetzenden Flußsäure richtet sich nach der Zahl der auszutauschenden Chloratome. Bei Verwendung von 1 Mol an ß-Ketotrihalogenmethyl-thioether der Formel (IV) muß pro auszutauschendem Chloratom mindestens 1 Mol an wasserfreier Flußsäure eingesetzt werden. Zweckmäßigerweise verwendet man jedoch einen Überschuß, so daß die Flußsäure gleichzeitig als Lösungsmittel fungieren kann. Im allgemeinen verwendet man beim Einsatz von 1 Mol an ß-Keto-trihalogenmethyl-thioether der Formel (IV) pro auszutauschendem Chloratom 10 bis 20 Mol Flußsäure. Ein größerer Überschuß ist jedoch nicht erschwerend für die Umsetzung.

Die Aufarbeitung des nach der Fluorierung anfallenden Reaktionsgemisches erfolgt nach üblichen Methoden. Im allgemeinen werden die Reaktionsprodukte durch Destillation isoliert.

Es ist jedoch auch möglich, das Reaktionsgemisch zunächst mit einem Lösungsmittel zu extrahieren, das mit

Flußsäure nur wenig mischbar ist. Derartige Lösungsmittel sind zum Beispiel Methylenchlorid, Chloroform, Tetrachlorkohlenstoff, Benzin, Benzol und Chlorbenzol.

Die nach der Extraktion erhaltene organische Phase wird gegebenenfalls nach vorherigem Waschen und Trocknen eingedampft und gegebenenfalls destilliert.

Die bei dem erfindungsgemäßen Verfahren weiterhin als Ausgangsstoffe benötigten Verbindungen sind durch die Formel (III) allgemein definiert. In dieser Formel haben $R^4$ und $R^5$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Verbindungen der Formel (III) sind allgemein bekannte Stoffe in der organischen Chemie.

Als Verdünnungsmittel können bei der Durchführung des erfindungsgemäßen Verfahrens praktisch alle inerten organischen Solventien verwendet werden. Vorzugsweise in Frage kommen aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether wie Diethyl- und Dibutylether, Tetrahydrofuran

und Dioxan, Ketone wie Aceton, Methylethyl-, Methylisopropyl- und Methylisobutylketon, Nitrile wie Acetonitril und Propionitril, Ester wie Essigsäuremethylester oder -ethylester, sowie Formamide wie Dimethylformamid.

In manchen Fällen kann auch Wasser als Verdünnungsmittel fungieren.

Als Säureakzeptoren können alle üblichen Säurebindemittel Verwendung finden. Vorzugsweise verwendbar sind Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diaza-bicyclo-[4,3,0]-non-5-en und 1,8-Diaza-bicyclo-[5,4,0]-undec-7-en.

An Stelle der oben genannten Säureakzeptoren können auch die in diesem Falle in entsprechendem Überschuß einzusetzenden Reaktanden der Formel (III) als Säurebindemittel fungieren.

Die Reaktionstemperatur kann innerhalb eines größeren Bereichs variiert werden. Im allgemeinen arbeitet man zwischen -20 und +100°C, vorzugsweise zwischen 0 und +50°C.

Das erfindungsgemäße Verfahren wird im allgemeinen bei Normaldruck durchgeführt.

Bei der Durchführung des erfindungsgemäßen Verfahrens setzt man auf 1 Mol an fluoriertem Thiokohlensäureesterfluorid der Formel (II) im allgemeinen 1 bis 2 Mol, vorzugsweise 1 bis 1,5 Mol einer Verbindung der Formel (III) sowie gegebenenfalls 1 bis 2 Mol oder auch einen größeren Überschuß an Säurebindemittel ein. Die Aufarbeitung erfolgt nach üblichen Methoden. Im allgemeinen geht man so vor, daß man das Reaktionsgemisch, gegebenenfalls nach vorherigem Versetzen mit einem mit Wasser nicht mischbaren organischen Solvens, mit Wasser wäscht, die organische Phase trocknet, einengt und den verbleibenden Rückstand destilliert oder in anderer Weise reinigt.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cir-

sium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-,

Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung von Cyperaceen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie

natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Beispiel 1

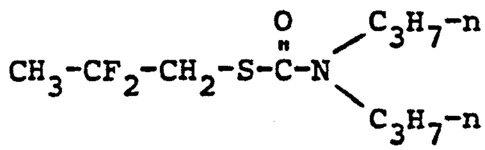

(I-1)

Zu einer Lösung von 15,8 g (0,1 Mol) 2,2-Difluorpropanthiol-kohlensäureesterfluorid in 100 ml Toluol wurden unter Rühren und Kühlung bei 20 - 28°C insgesamt 20,2 g (0,2 Mol) Di-n-propylamin zugetropft. Das Reaktionsgemisch wurde weitere 30 Minuten bei Raumtemperatur gerührt und dann mit Wasser gewaschen. Nach dem Trocknen der organischen Phase über Natriumsulfat wurde das Lösungsmittel unter vermindertem Druck abgezogen und der verbleibende Rückstand einer fraktionierten Vakuumdestillation unterworfen. Man erhielt auf diese Weise 21 g (83 % der Theorie) an N,N-Di-n-propyl-(2,2-difluor-propyl)-thiolcarbamidsäureester in Form einer Flüssigkeit vom Siedepunkt 125°C/12 mbar.

Nach der im Beispiel 1 angegebenen Methode wurden auch in der folgenden Tabelle 3 formelmäßig aufgeführten Stoffe hergestellt.

Tabelle 3

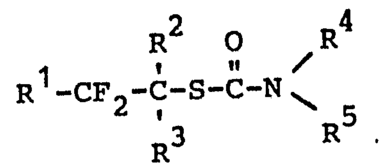

(I)

| Beispiel Nr. | $R^1$ | $R^2$ | $R^3$ | $R^4$ | $R^5$ | Physik. Konst. |
|---|---|---|---|---|---|---|
| 2 | $CH_3$ | H | H | H | $CH_3$ | Kp 113°C/12 mbar |
| 3 | $CH_3$ | H | H | $C_2H_5$ | $C_2H_5$ | Kp 107°C/12 mbar |
| 4 | $CH_3$ | H | H | $i\text{-}C_3H_7$ | $i\text{-}C_3H_7$ | Kp 113-115°C/12 mbar |
| 5 | $CH_3$ | H | H | $i\text{-}C_4H_9$ | $i\text{-}C_4H_9$ | Kp 130-131°C/12 mbar |
| 6 | $CH_3$ | H | H | $-(CH_2)_4-$ | | Kp 145°C/20 mbar |
| 7 | $CH_3$ | H | H | $-(CH_2)_5-$ | | Kp 135-136°C/15 mbar |
| 8 | $CH_3$ | H | H | $-(CH_2)_6-$ | | Kp 156°C/19 mbar |
| 9 | $CH_3$ | H | H | $-(CH_2)_7-$ | | Kp 98-100°C/0,2 mbar |
| 10 | $CH_3$ | H | H | $i\text{-}C_3H_7$ | $(CH_3)_3C\text{-}CH_2$ | Kp 120°C/18 mbar |
| 11 | $CH_3$ | H | H | $-(CH_2)_2\text{-}S\text{-}(CH_2)_2-$ | | Kp 108-110°C/0,15 mbar |
| 12 | $CH_3$ | H | H | $-(CH_2)_2\text{-}SO_2\text{-}(CH_2)_2-$ | | Fp 104-105°C |
| 13 | $CH_3$ | H | H | $-(CH_2)_2\text{-}CH(CH_3)\text{-}(CH_2)_2-$ | | Kp 151-152°C/20 mbar |

<u>Tabelle 3</u> (Fortsetzung)

| Beispiel Nr. | $R^1$ | $R^2$ | $R^3$ | $R^4$ | $R^5$ | Physik. Konst. |
|---|---|---|---|---|---|---|
| 14 | $CH_3$ | H | H | | 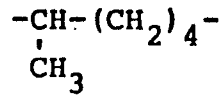 | Kp 92-95°C/0,2 mbar |
| 15 | $CH_3$ | H | H | | 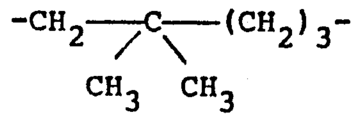 | Kp 95-100°C/0,12 mbar |
| 16 | $CH_3$ | H | H | | 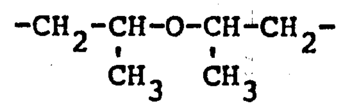 | Kp 89-91°C/0,17 mbar |
| 17 | $CH_3$ | $CH_3$ | H | $C_2H_5$ | $C_2H_5$ | Kp 113-115°C/18 mbar |
| 18 | $CH_3$ | $CH_3$ | H | $i-C_3H_7$ | $i-C_3H_7$ | Kp 118-121°C/20 mbar |
| 19 | $CH_3$ | $CH_3$ | H | $-(CH_2)_4-$ | | Kp 142-144°C/18 mbar |
| 20 | $CH_3$ | $CH_3$ | H | $-(CH_2)_5-$ | | Kp 148-149°C/18 mbar |
| 21 | $CH_3$ | $CH_3$ | H | $-(CH_2)_6-$ | | Kp 108-110°C/0,2 mbar |
| 22 | $CH_3$ | $CH_3$ | H | $-(CH_2)_2-S-(CH_2)_2-$ | | Fp 53-54°C |
| 23 | $CH_3$ | $CH_3$ | H | $-(CH_2)_2-SO_2-(CH_2)_2-$ | | Fp 104-105°C |
| 24 | $CH_3$ | $CH_3$ | H | | 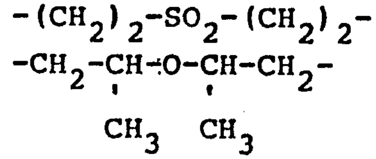 | Kp 96-99°C/0,12 mbar |
| 25 | $CH_3$ | $CH_3$ | H | | 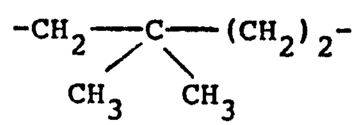 | Kp 92-95°C/0,2 mbar |

Tabelle 3 (Fortsetzung)

| Beispiel Nr. | $R^1$ | $R^2$ | $R^3$ | $R^4$ | $R^5$ | Physik. Konst. |
|---|---|---|---|---|---|---|
| 26 | $CH_3$ | $CH_3$ | H | | $-(CH_2)_2-CH-(CH_2)_2-$<br>$CH_3$ | Kp 154-156°C/20 mbar |
| 27 | $CH_3$ | $CH_3$ | H | | $-CH-(CH_2)_4-$<br>$CH_3$ | Kp 95-98°C/0,15 mbar |
| 28 | $CH_3$ | $CH_3$ | H | | 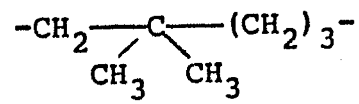 | Kp 95-98°C/0,18 mbar |
| 29 | $CH_3$ | $CH_3$ | H | | $-CH-(CH_2)_3-CH-$<br>$CH_3$ $CH_3$ | Kp 90°C/0,15 mbar |
| 30 | $n-C_3H_7$ | $C_2H_5$ | H | | $-(CH_2)_4-$ | $n_D^{20}$ = 1,4808 |
| 31 | $n-C_3H_7$ | $C_2H_5$ | H | | $-(CH_2)_5-$ | $n_D^{20}$ = 1,4803 |
| 32 | 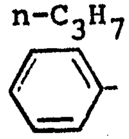 | H | H | | $-(CH_2)_4-$ | $n_D^{20}$ = 1,5414 |
| 33 | 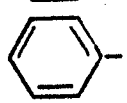 | H | H | | $-(CH_2)_5-$ | $n_D^{20}$ = 1,5362 |

**Beispiel 34**

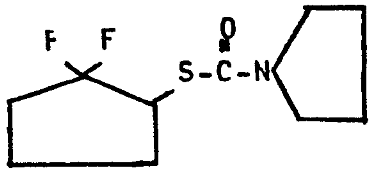

Die Verbindung zeigt im 60 MHz H-NMR-Spektrum folgende Signale ($CDCl_3$):

1,6 - 2,5 ppm (Multiplett) Protonen der Ringmethylen-Gruppen
3,4 ppm (Multiplett) Protonen der zu N $\alpha$-ständigen $CH_2$-Gruppen
4,1 ppm (Multiplett) Proton der Ringmethin-Gruppe

**Beispiel 35**

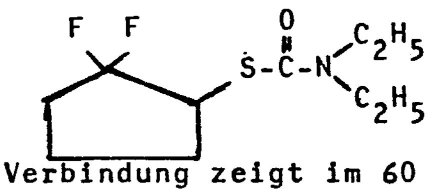

Die Verbindung zeigt im 60 MHz H-NMR-Spektrum folgende Signale:

1,17 ppm (Triplett) Protonen der $CH_3$-Gruppen
1,6 - 2,5 ppm (Multiplett) Protonen der Ringmethylen-Gruppen
3,37 ppm (Quartett) Protonen der $CH_2$-Gruppen der Ethyl-Gruppen
4,1 ppm (Multiplett) Protonen der Ringmethin-Gruppe

Beispiel 36

$$CH_3-CF_2-CH_2-S-\underset{\overset{\|}{O}}{C}-F \qquad (II - 1)$$

In einem Reaktionsgefäß aus rostfreiem Stahl wurden 200 ml wasserfreie Flußsäure vorgelegt. Bei einer Temperatur von -10°C ließ man innerhalb von 30 Minuten 200 g (1,05 Mol) Acetonyl-dichlorfluormethyl-thioether unter Rühren zutropfen, wobei sofort eine Chlorwasserstoffentwicklung einsetzte. Man erwärmte das Reaktionsgemisch bis auf 0°C und rührte bei dieser Temperatur noch so lange nach, bis praktisch keine Gasentwicklung mehr beobachtet wurde. Zur anschließenden Aufarbeitung wurde das Reaktionsgemisch fraktioniert destilliert. Man erhielt auf diese Weise 109 g (66 % der Theorie) an 2,2-Difluorpropanthiolkohlensäureesterfluorid.

Kp. 53°C/100 mbar

$n_D^{20} = 1,3907$

Nach der im Beispiel 3 6 angegebenen Methode wurden auch die in der folgenden Tabelle 4 formelmäßig aufgeführten Stoffe hergestellt.

Tabelle 4

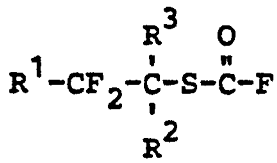

(II)

| Beispiel Nr. | $R^1$ | $R^2$ | $R^3$ | Siedepunkt/Brechungsindex |
|---|---|---|---|---|
| 37 | $CH_3-$ | $CH_3-$ | H | 59°C/100 mbar<br>$n_D^{20}$ = 1,3950 |
| 38 | $CH_3-$ | $C_2H_5-$ | H | 76°C/100 mbar<br>$n_D^{20}$ = 1,4042 |
| 39 | 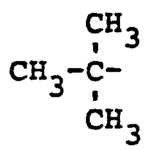 | H | H | 66°C/20 mbar<br>$n_D^{20}$ = 1,4160 |
| 40 | $n-C_3H_7-$ | $C_2H_5-$ | H | 75°C/19 mbar<br>$n_D^{20}$ = 1,4148 |
| 41 | $CH_3-$ | $CH_3-$ | $CH_3-$ | 72°C/50 mbar<br>$n_D^{20}$ = 1,4128 |
| 42 | $-(CH_2)_3-$ | | H | 55 - 57 °C / 16 mbar |

Beispiel 43

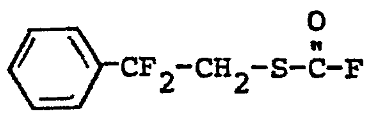

(II - 8)

In einem Reaktionsgefäß aus rostfreiem Stahl wurden 400 ml wasserfreie Flußsäure vorgelegt. Bei einer Temperatur von -8°C ließ man innerhalb von einer Stunde 530 g (2,09 Mol) (Fluordichlormethyl-mercapto-methyl)-phenyl-keton unter Rühren zutropfen. Die dabei einsetzende Chlorwasserstoff-Entwicklung klang nach etwa einer Stunde ab und wurde durch vierstündiges Nachrühren bei Raumtemperatur ganz beendet. Zur anschließenden Aufarbeitung wurde das Reaktionsgemisch einer fraktionierten Destillation unter vermindertem Druck unterworfen. Man erhielt auf diese Weise 342 g (74 % der Theorie) an ß-Phenyl-ß,ß-difluor-ethanthiol-kohlensäureesterfluorid.

Kp = 106°C/18 mbar
$n_D^{20}$ = 1,4891

Herstellung des Vorproduktes der Formel

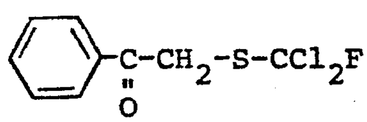

(IV-1)

240 g (2 Mol) Acetophenon wurden in 300 ml Chloroform gelöst und nach Zugabe von 3 ml Ethanol und 109 g (0,64 Mol) Fluordichlormethan-sulfenchlorid bis zur Rückflußtemperatur aufgeheizt. Innerhalb von 30 Minuten ließ man noch 400 g (2,36 Mol) an Fluordichlormethan-sulfenchlorid zutropfen und erhitzte dann 5 Stunden unter Rückfluß. Zur anschließenden Aufarbeitung wurde das Reaktionsgemisch einer fraktionierten Destillation unter vermindertem Druck unterworfen. Nach Vorläufen von unverändertem Ausgangsmaterial erhielt man 126 g an (Fluordichlormethyl-mercapto-methyl)-phenyl-keton in Form einer Flüssigkeit vom Siedepunkt 128°C/0,4 mbar.

Brechungsindex: $n_D^{20}$ = 1,5653

Die Ausbeute errechnet sich zu 87 %, bezogen auf umgesetztes Acetophenon.

Das Reaktionsprodukt kann auch ohne zusätzliche Reinigung nach Abtrennung des unveränderten Acetophenons für die Fluorierung mit Flußsäure eingesetzt werden.

Beispiel 44

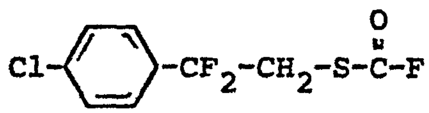

(II-9)

In einem Reaktionsgefäß aus rostfreiem Stahl wurden 300 ml wasserfreie Flußsäure vorgelegt. Bei einer Temperatur von -8°C ließ man innerhalb von einer Stunde 370 g (1,29 Mol) (4-Chlor-phenyl)-(fluordichlormethylmercaptomethyl)-keton unter Rühren zutropfen. Nach dem Abklingen der zunächst sehr zügig einsetzenden Chlorwasserstoff-Entwicklung ließ man die Temperatur der Reaktionsmischung langsam auf Raumtemperatur ansteigen und rührte bis zur Beendigung der Chlorwasserstoff-Entwicklung. Zur anschließenden Aufarbeitung wurde das Reaktionsgemisch einer fraktionierten Destillation unter vermindertem Druck unterworfen. Man erhielt auf diese Weise 211 g (64,1 % der Theorie) an ß-(4-Chlor-phenyl)-ß,ß-difluorethanthiolkohlensäureester-fluorid in Form einer Flüssigkeit.

Kp = 126°C/16 mbar

$n_D^{20}$ = 1,5083

Beispiel 45

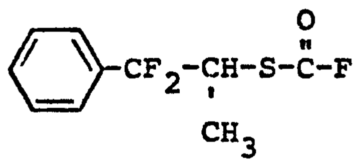

(II-10)

Nach den in den Beispielen 43 und 44 angegebenen Methoden wurde auch das (2-Phenyl-2,2-difluor-1-methyl)-ethanthiol-kohlensäureester-fluorid hergestellt.

Kp = 107°C/14 mbar
$n_D^{20}$ = 1,4839

Beispiel 46

Synthese des Ausgangsproduktes der Formel

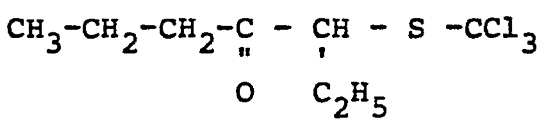

(IV-2)

Ein Gemisch aus 171 g (1,5 Mol) Di-n-propylketon und 186 g (1 Mol) Perchlormethylmercaptan wurde 14 Stunden auf 100°C erhitzt. Dabei beobachtete man eine schwache, aber regelmäßige Chlorwasserstoff-Entwicklung. Zur anschließenden Aufarbeitung wurde das Reaktionsgemisch einer fraktionierten Destillation unter vermindertem Druck unterworfen. Man erhielt auf diese Weise 80 g eines Produktes, das im wesentlichen aus dem ß-Keto-

trichlormethyl-thioether der Formel (IV-2) bestand, jedoch noch durch die Substanz der Formel

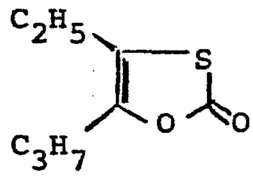

verunreinigt war.

Kp = 109°C/0,1 mbar
$n_D^{20}$ = 1,5156

Bei der Umsetzung mit der Flußsäure bildet sich aus dem oben angegebenen Substanzgemisch die im Beispiel 40 beschriebene Verbindung.

In dem nachstehend beschriebenen biologischen Test wurde die folgende Verbindung als Vergleichssubstanz eingesetzt:

(A) = 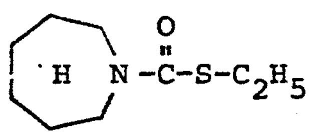

Hexahydro-1-H-azepin-1-carbamidsäure-thiolethylester (bekannt aus US-PS 3 198 786).

## Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigte die Verbindung gemäß Beispiel 19 bei der Bekämpfung von Cyperus und Setaria in Mais und Baumwolle eine deutlich bessere selektive herbizide Wirksamkeit als die Vergleichssubstanz (A).

## Patentansprüche

1. Fluorierte Thiolcarbamate der Formel

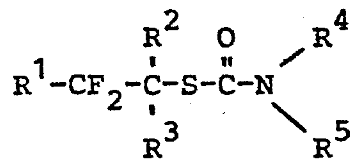

(I)

in welcher

$R^1$ für Alkyl, Cycloalkyl oder gegebenenfalls substituiertes Aryl steht,

$R^2$ und $R^3$ unabhängig voneinander für Wasserstoff oder Alkyl stehen, oder

$R^1$ und $R^2$ gemeinsam für eine Alkylenkette stehen und

$R^4$ und $R^5$ unabhängig voneinander für Wasserstoff, Alkyl, Alkenyl, Alkinyl, Alkoxyalkyl, Alkylthioalkyl, Cycloalkyl oder einen gesättigten oder ungesättigten gegebenenfalls substituierten heterocyclischen Ring mit 5 bis 8 Ringgliedern stehen,

oder

$R^4$ und $R^5$ gemeinsam mit dem angrenzenden Stickstoffatom für einen gegebenenfalls substituierten gesättigten oder ungesättigten heterocyclischen Ring mit 5 bis 8 Ringgliedern stehen.

2. Fluorierte Thiolcarbamate der Formel (I), in denen

$R^1$ für geradkettiges oder verzweigtes Alkyl mit 1 bis 12 Kohlenstoffatomen, Cycloalkyl mit 3 bis 8 Kohlenstoffatomen oder für Phenyl steht, welches ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen, Alkylthio mit 1 bis 4 Kohlenstoffatomen, Halogenalkylthio mit 1 bis 4 Kohlenstoffatomen und 1 bis 5 Halogenatomen und/oder Nitro,

$R^2$ und $R^3$ unabhängig voneinander für Wasserstoff oder geradkettiges oder verzweigtes Alkyl mit 1 bis 4 Kohlenstoffatomen stehen oder

$R^1$ und $R^2$ gemeinsam für eine Alkylenkette mit 3 bis 5 Kohlenstoffatomen stehen und

$R^4$ und $R^5$ unabhängig voneinander für Wasserstoff, Alkyl mit 1 bis 4 Kohlenstoffatomen, Alkenyl mit 2 bis 4 Kohlenstoffatomen, Alkinyl mit 2 bis 4 Kohlenstoffatomen, Alkoxyalkyl mit 1 bis 4 Kohlenstoffatomen im Alkoxyteil und 1 bis 4 Kohlenstoffatomen im Alkylteil, Alkylthioalkyl

mit 1 bis 4 Kohlenstoffatomen im Alkylthioteil und 1 bis 4 Kohlenstoffatomen im Alkylteil, Cycloalkyl mit 3 bis 8 Kohlenstoffatomen oder für einen gesättigten oder ungesättigten, gegebenenfalls durch Halogen, Alkyl mit 1 bis 4 Kohlenstoffatomen, Alkoxy mit 1 bis 4 Kohlenstoffatomen und/oder Phenyl substituierten heterocyclischen Ring stehen, der 5 bis 8 Ring-Glieder und 1 bis 3 Heteroatome bzw. Heteroatom-Gruppen, wie Sauerstoff, Stickstoff, Schwefel, $SO_2$, SO und/oder NR, worin R für Wasserstoff oder Alkyl mit 1 bis 4 Kohlenstoffatomen steht, enthält,

oder

$R^4$ und $R^5$ gemeinsam mit dem angrenzenden Stickstoffatom für einen gesättigten oder ungesättigten, gegebenenfalls durch Halogen, Alkyl mit 1 bis 4 Kohlenstoffatomen, Alkoxy mit 1 bis 4 Kohlenstoffatomen und/oder Phenyl substituierten Heterocyclus stehen, der 5 bis 8 Ringglieder und 1 bis 3 Heteroatome bzw. Heteroatom-Gruppen, wie Sauerstoff, Stickstoff, Schwefel, SO, $SO_2$ und/oder NR, worin R für Wasserstoff oder Alkyl mit 1 bis 4 Kohlenstoffatomen steht, enthält.

3. Verfahren zur Herstellung von fluorierten Thiolcarbamaten der Formel

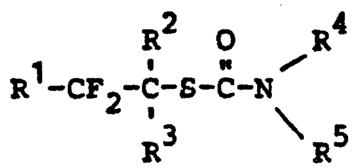

(I)

in welcher

$R^1$ für Alkyl, Cycloalkyl oder gegebenenfalls substituiertes Aryl steht,

$R^2$ und $R^3$ unabhängig voneinander für Wasserstoff oder Alkyl stehen, oder

$R^1$ und $R^2$ gemeinsam für eine Alkylenkette stehen und

$R^4$ und $R^5$ unabhängig voneinander für Wasserstoff, Alkyl, Alkenyl, Alkinyl, Alkoxyalkyl, Alkylthioalkyl, Cycloalkyl oder einen gesättigten oder ungesättigten gegebenenfalls substituierten heterocyclischen Ring mit 5 bis 8 Ringgliedern stehen,

oder

$R^4$ und $R^5$ gemeinsam mit dem angrenzenden Stickstoffatom für einen gegebenenfalls substituierten gesättigten oder ungesättigten heterocyclischen Ring mit 5 bis 8 Ringgliedern stehen,

dadurch gekennzeichnet, daß man fluorierte Thiokohlensäureesterfluoride der Formel

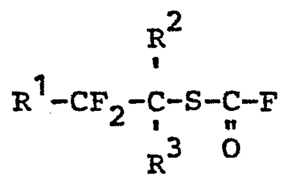

(II)

in welcher

$R^1$, $R^2$ und $R^3$ die oben angegebene Bedeutung haben,

mit Verbindungen der Formel

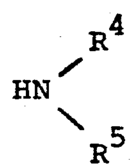

(III)

in welcher

$R^4$ und $R^5$ die oben angegebene Bedeutung haben,

gegebenenfalls in Gegenwart eines Verdünnungsmittels sowie gegebenenfalls in Gegenwart eines Säurebindemittels umsetzt.

4. Herbizide Mittel, gekennzeichnet, durch einen Gehalt an mindestens einem fluorierten Thiolcarbamat der Formel (I).

5. Verfahren zur Bekämpfung von Unkräutern, dadurch gekennzeichnet, daß man fluorierte Thiolcarbamate der Formel (I) auf die Unkräuter und/oder deren Lebensraum ausbringt.

6. Verwendung von fluorierten Thiolcarbamaten der Formel (I) zur Bekämpfung von Unkräutern.

7. Verfahren zur Herstellung von herbiziden Mitteln, dadurch gekennzeichnet, daß man fluorierte Thiolcarbamate der Formel (I) mit Streckmitteln und/oder oberflächenaktiven Stoffen vermischt.

8. Fluoriertes Thiolcarbamat der Formel

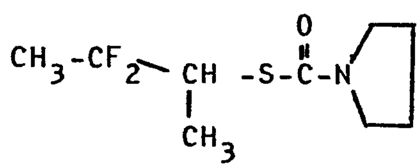
.

9. Fluoriertes Thiolcarbamat der Formel

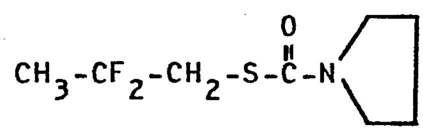
.

10. Fluoriertes Thiolcarbamat der Formel

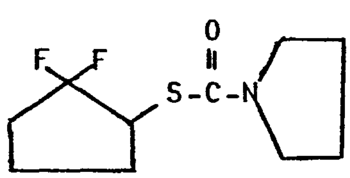
.